# EUROPEAN PATENT SPECIFICATION

(11) **EP 3 122 296 B1**
(45) Date of publication and mention of the grant of the patent: **17.10.2018**
(21) Application number: 15793441.5
(22) Date of filing: 04.05.2015
(51) Int. Cl.: A61F 9/00, A61M 35/00

(54) **OPHTHALMIC TREATMENT SOLUTION DELIVERY DEVICES AND DELIVERY AUGMENTATION METHODS**
OPHTHALMISCHE BEHANDLUNGSLÖSUNGSABGABEVORRICHTUNGEN UND ABGABEVERSTÄRKUNGSVERFAHREN
DISPOSITIFS D'ADMINISTRATION DE SOLUTION DE TRAITEMENT OPHTALMIQUE ET PROCÉDÉS D'AUGMENTATION D'ADMINISTRATION

(30) Priority: 12.05.2014 US 201414275192
(43) Date of publication of application: 01.02.2017
(60) Divisional application: 18182071.3
(73) Proprietor: CXL Ophthalmics, LLC, Encinitas, California 92024 (US)
(72) Inventor: RUBINFELD, Roy S., Bethesda, Maryland 20817 (US); KORTEWEG, Wayne, N. Stonington, Connecticut 06359 (US)
(74) Representative: FRKelly
(86) International application number: PCT/US2015/029011
(87) International publication number: WO 2015/175250

(56) References cited:
- WO-A1-00/63079
- WO-A1-2013/148895
- WO-A1-2013/148896
- WO-A1-2013/148896
- WO-A1-2013/158661
- US-A- 4 098 728
- US-A1- 2001 022 063
- US-A1- 2013 331 768

## Description

### BACKGROUND

Collagen cross-linking is a treatment for multiple ophthalmic disorders. In some cases, collagen cross-linking may also be combined with other treatments to improve corneal strength or optical refraction, such as corneal ring segment inserts, topography-guided laser, and the like. Corrective lenses are normally required after these treatments for weakened corneas, but with smaller, more normalized prescriptions. Increased corneal symmetry allows for more comfortable contact lens wear, often of daily disposable lenses. Collagen cross-linking limits deterioration of vision, increases unaided and uncorrected vision, and may reduce the need for corneal transplantation. Collagen cross-linking may also have a role in stabilizing and "locking in" refractive effects of other procedures.

WO 2013/148896 discloses a preparation device and method for buffing the corneal epithelium allows better penetration of the epithelium by riboflavin solution when subsequently applied to the eye; and a riboflavin solution for use in corneal strengthening treatment and the like has a higher concentration of riboflavin and may include other components for increasing corneal cross-linking.

### SUMMARY

Disclosed herein is a method of applying an ophthalmic composition to an eye, comprising applying the ophthalmic composition after preparing the epithelium of an eye for more effective treatment. The method comprises preparing the epithelium of the eye using a delivery system as is claimed by rubbing or contacting the eye with a first eye surface preparation sponge device, so as to manipulate or "buff" the tissue of the epithelium.

The method can comprise preparing the epithelium of the eye by rubbing the eye with the first eye surface preparation sponge device in a circular pattern. The method can comprise removing lipids, mucus and microvilli. The first eye surface preparation sponge device comprises a handle with a sponge attached to one end of the handle for contacting the eye. In some embodiments, the first eye surface preparation sponge is dry or pre-wetted with an ophthalmic composition. In some embodiments, the sponge is pre-wetted with a riboflavin composition, artificial tears, or a combination thereof. In some embodiments, use of the first eye surface preparation sponge device to prepare the epithelium markedly increases the permeability of the epithelium to medications or ophthalmic solutions applied to the surface of the epithelium after preparation or polishing with the buffing or polishing sponge.

The method further comprises applying the ophthalmic composition with a second, loading sponge device. In some embodiments, the method further comprises placing the second loading sponge device over the eye to act as a depot or reservoir for holding the ophthalmic composition or solution in contact with the eye surface over an area where the solution needs to be absorbed into the eye.

In certain embodiments, the second loading sponge device comprises a circular, disc-shaped loading sponge which peri- or pre-operatively covers all or a portion of the eye surface. In some embodiments, the size of the loading sponge does not exceed the eye surface. In some embodiments, the diameter of the sponge is about 3 mm. to about 12 mm. In some embodiments, the second loading sponge device has a diameter of around 11.5 mm and may be pre-formed to follow the curvature of most or all of the exposed, curved surface of the eye or may be of flexible material designed to adopt the shape of the exposed, curved surface of the eye. In some embodiments, the second loading sponge device or loading sponge may be presaturated with an ophthalmic solution. In some embodiments, the second sponge device may be placed on the eye surface and solution can be dripped onto the sponge device after placement. In some embodiments, the ophthalmic solution may comprise 0.2% to 10.0% by weight riboflavin in an aqueous carrier, and optionally, sodium iodide, catalase, artificial tears, or any combinations thereof.

The first eye surface preparation sponge device is for use in manipulation of tissue on the surface of an eye prior to application of the second loading sponge device. The first, eye surface preparation sponge device comprises a preparation sponge having a tissue preparation surface shaped for manipulating and rubbing across the surface of an eye. In some embodiments, the first eye surface preparation sponge has little or no risk of disrupting or perforating the epithelium. The first eye surface preparation sponge device comprises a handle operatively connected to the preparation sponge.

The tissue preparation surface of the first eye surface preparation sponge is generally rounded with no sharp edges, when wetted.

In some embodiments, the preparation sponge is made of a cellulose sponge material. In some embodiments, the preparation sponge is made of polyvinyl acetate (PVA) sponge material. In some embodiments, the preparation sponge is wetted with an ophthalmic solution prior to use in preparation of the eye surface. In some embodiments, the ophthalmic solution is 0.2% to 10.0% by weight riboflavin in an aqueous carrier, and optionally, sodium iodide, catalase, artificial tears, or any combinations thereof.

Use of the first, eye surface preparation sponge device followed by the second loading sponge device may augment or enhance delivery of any ophthalmic treatment solution to the eye for use in photochemical treatment of the cornea or for other types of treatment, such as application of eye medications such as glaucoma medications or anti-inflammatory medications such as steroids. In certain embodiments, the treatment solution is a riboflavin solution.

Disclosed herein, in certain embodiments, is a delivery system that improves the passage or penetration of riboflavin or other ophthalmic drugs through the epithelial barrier to avoid the surgical complications that arise from de-epithelialization, which can result in more disruption of the tissue than is necessary for the procedure to be effective. The reduction in patient discomfort combined with more rapid restoration of visual acuity make a trans-epithelial procedure better for the patient. In some embodiments, the first eye surface preparation and second loading sponge devices are sterile. In some embodiments, the first eye surface preparation sponge is part circular shape when dry and is configured to absorb liquid and expand to be generally rounded with no sharp edges when wetted. In some embodiments, the the first eye surface preparation sponge is spherical or part-spherical in its dry condition. The first eye surface preparation sponge may be of cellulose, PVA, or urethane sponge material. The first eye surface preparation sponge device or preparation sponge has little or no risk of disrupting or perforating the epithelium, resulting in as little disruption of the epithelium as possible while still increasing penetration of the riboflavin or other solution into the deeper layers of the cornea without disrupting or causing any significant epithelial defects in the corneal surface.

The first eye surface preparation sponge device is of absorbent, fast wicking sponge material designed to enhance epithelial permeability by gently removing lipids, mucus, and dead surface epithelial cells. In some embodiments, the preparation sponge is packaged along with a blunt plastic shaft or other tool to assist in "twirling" this sponge around on the cornea or sclera or other parts of the eye to manipulate the tissue to be treated in an effort to enhance penetration of the riboflavin or other solution into the eye.

Further disclosed herein is a method of using a first eye surface preparation sponge device as disclosed herein to improve the penetration of riboflavin or other ophthalmic drugs through the epithelial barrier to avoid the surgical complications that arise from de-epithelialization, which result in more disruption of the tissue than is necessary for the procedure to be effective. The reduction in patient discomfort combined with more rapid restoration of visual acuity make a trans-epithelial procedure better for the patient. In some embodiments, the first eye surface preparation sponge is rubbed gently over the surface of the eye in a circular pattern after application of a topical anesthetic, in order to prepare the epithelium for improved penetration of riboflavin or other solutions. In some embodiments, the method removes lipids, mucus, microvilli and other natural barriers to riboflavin or other ocular solutions loading into the deeper ocular tissues. In some embodiments, it is possible that this preparation may produce very fine micro-abrasion to allow the riboflavin solution to penetrate through the epithelium into the cornea more easily, but this is not essential and may not occur in all cases. In some embodiments, the method has little or no risk of disrupting or perforating the epithelium resulting in as little disruption of the epithelium as possible while still increasing penetration of the riboflavin or other solution into the deeper layers of the cornea. The method further comprises applying an ophthalmic solution (e.g., a riboflavin solution) to the eye
through a second, loading sponge device placed over the eye to act as a depot or reservoir for the ophthalmic solution, since the tear film itself is only about 5 microns thick, which offers only a very small reservoir of riboflavin solution. This also moisturizes the corneal surface.

In various embodiments, the sponge device is used in one or more microsurgical ophthalmic procedures for tissue manipulation and management of fluids. In other embodiments, the sponge device is used in one or more microsurgical ophthalmic procedures for the management of one or more fluids. In some embodiments, the sponge device is placed on the cornea to moisten the cornea during one or more microsurgical procedures.

The above devices and methods may be used in various microsurgical ophthalmic procedures, including preparation of the epithelium for loading of one or more fluids through the epithelium into the eye for photochemical or other treatment.

Other features and advantages of the present disclosure will become more readily apparent to those of ordinary skill in the art after reviewing the following detailed description and accompanying drawings.

### BRIEF DESCRIPTION OF THE DRAWINGS

The details of the present disclosure, both as to its structure and operation, may be gleaned in part by study of the accompanying drawings, in which like reference numerals refer to like parts, and in which:
FIG. 1 exemplifies an enlarged perspective view of one embodiment of a first eye surface preparation sponge device in a dry condition for use in preparing the corneal epithelium for subsequent application of an ocular treatment solution to the eye;
FIG. 2 is a cross section on the lines 2-2 of FIG. 1;
FIG. 3A is a top plan view of a spear shaped sponge attached to the handle of FIG. 1 and 2 prior to cutting the sponge into the rounded shape of FIG. 1 and 2, according to one example of a method of making the preparation sponge device of FIG. 1 and 2;
FIG. 3B is a top plan view of a sponge blank attached to the handle of FIG. 1 and 2 prior to cutting the sponge into the rounded shape of FIG. 1 and 2, according to another example of a method of making the preparation sponge device of FIG. 1 and 2
FIG. 4 is a cut away top perspective view of the tissue preparation sponge device of FIG. 1 to 3, showing the sponge head with part of the handle cut away, with the sponge head in a dry condition;
FIG. 5 is a side elevation view of the preparation sponge device of FIG. 1 to 4, with the handle partially cut away as in FIG. 2 and 4;
FIG. 6 is a top end perspective view of the preparation sponge device of FIGS. 1 to 5, with the handle partially cut away as in FIGS. 4 and 5;
FIGS. 7 to 9 are cut away views of the preparation sponge device corresponding to the views of FIGS. 4 to 6 but with the sponge head in a wet, expanded condition;
FIG. 10 illustrates an exemplary use of the sponge of FIG. 1 to 9 to manipulate the corneal surface in a swirl action;
FIGS. 11A to 11C are top plan views illustrating some other embodiments of the buffing or polishing sponge device with a sponge head being generally rounded with no sharp edges;
FIG. 12 is a top plan view illustrating another embodiment of the buffing or polishing sponge device head; being generally rounded with no sharp edges;
FIG. 13 is a perspective view illustrating an embodiment of a second loading sponge device placed on an eye for holding and loading of an ophthalmic treatment solution;
FIG. 14 is a vertical cross-sectional view illustrating the second loading sponge device of FIG. 13 following the curvature of most of the eye surface, with details of the eye itself omitted;
FIG. 15 is a perspective view similar to FIG. 13 illustrating a modified embodiment of a round loading sponge; and
FIG. 16 illustrates tabulated examples of corneal saturation or loading times (in minutes) for a 0.1% riboflavin solution and a 0.5% riboflavin solution when the epithelium is prepared or manipulated with the sponge device of FIGS. 1 to 9 and one of the solution holding or loading sponges of FIG. 13 to 15 is used to load the riboflavin treatment solution.

### DETAILED DESCRIPTION

Certain embodiments as disclosed herein provide for ocular treatment solution delivery devices and delivery augmentation methods. For example, according to embodiments of the disclosure, devices and methods are provided for preparing the corneal epithelium for better penetration of riboflavin solutions or other ophthalmic solutions into the cornea.

A popular treatment of corneal diseases, including keratoconus, post-LASIK ectasia, and pellucid marginal degeneration, involves the removal of the epithelium followed by administration of a riboflavin solution and irradiation by ultraviolet-A light. Riboflavin acts as a photosensitizer and facilitates the cross-linking of stromal collagen fiber which prevents further disease progression. However, the removal of the epithelium carries numerous risks for the patient, including post-operative pain, infection risk, delayed wound healing, corneal perforation, stromal haze, and herpetic keratitis. Therefore, it would be safer to treat the patient without having to surgically remove the epithelium. Unfortunately, there are many obstacles to this approach because the intact epithelium prevents the cornea from rapidly and conveniently absorbing the riboflavin solution. This invention discloses novel devices and methods for allowing convenient treatment of corneal diseases with medications or with ophthalmic solutions such as riboflavin solutions without removing the epithelium, and may increase the effectiveness of such treatments.

After reading this description it will become apparent to one skilled in the art how to implement the disclosure in various alternative embodiments and alternative applications. However, although various embodiments of the present disclosure will be described herein, it is understood that these embodiments are presented by way of example only, and not limiting. As such, this detailed description of various alternative embodiments should not be construed to limit the scope or breadth of the present disclosure as set forth in the appended claims.

The time period for sufficient riboflavin to penetrate into the cornea when the solution is applied in drops to the eye without removal or treatment of the epithelium layer can be one to three hours. This is a problem for both patients undergoing treatment and for surgeons, in view of the extended time period needed. In some embodiments described below, eye surface preparation or manipulation reduces the initial time needed for an ophthalmic solution (e.g., a riboflavin solution or other ophthalmic solution) to penetrate sufficiently into the cornea in as little as seven to eleven minutes without requiring removal of the epithelium, significantly reducing patient discomfort and the time needed to complete the treatment.

Manipulation of the epithelium prepares it for improved penetration of the epithelium by the ophthalmic solution (e.g., a riboflavin solution), without having to remove the epithelium altogether. The cornea absorbs the riboflavin well until the corneal stroma is sufficiently loaded.

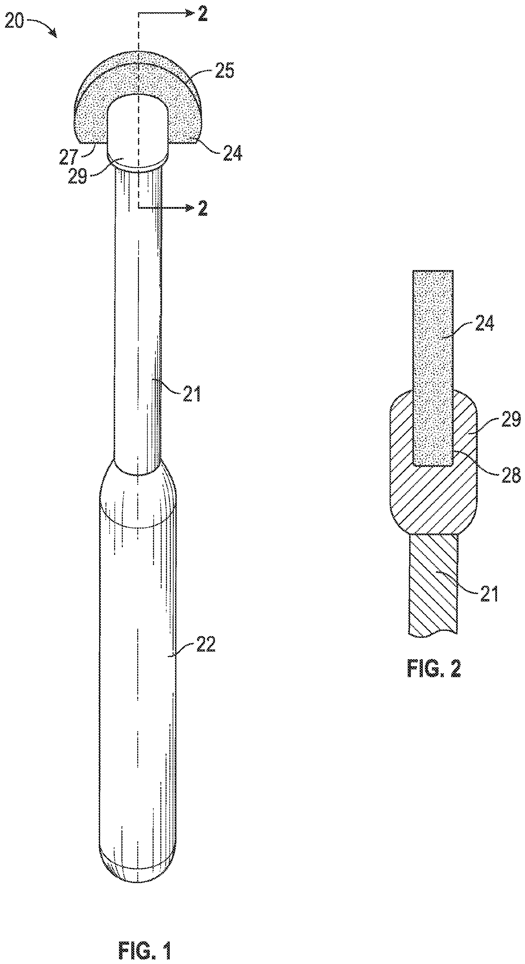

FIGS. 1 to 10 illustrate an exemplary device and method for improving the penetration of riboflavin or other ophthalmic solutions or fluids through the epithelial barrier, while avoiding the surgical complications that arise from deepithelialization. The reduction in patient discomfort, the more rapid restoration of visual acuity, and the reduced risk of infection or stromal haze make a trans epithelial procedure better for the patient.

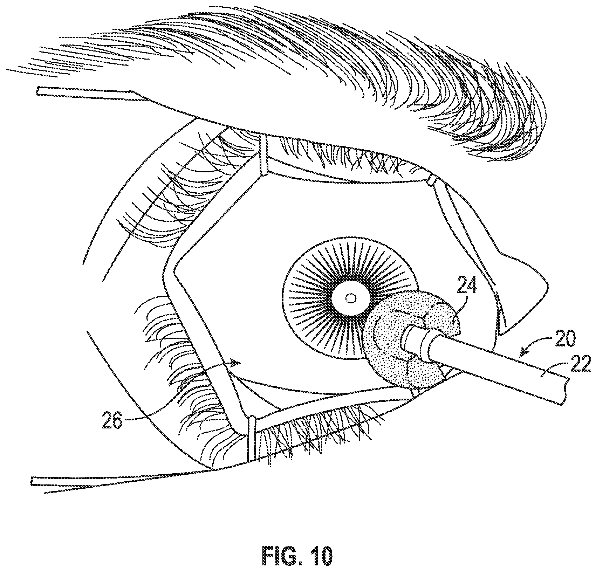

In some embodiments, a first eye surface preparation preparation sponge device 20 as illustrated in FIG. 1-9 is rubbed gently over the surface of the eye 26 after application of a topical anesthetic, as illustrated in FIG. 10. The sponge may be rubbed gently in a circular or swirling manner, in order to manipulate the eye surface, in other words to "buff" or "polish" the eye to a dull sheen. The first eye surface preparation sponge device 20 comprises an elongate handle or shaft 21 with a gripping portion 22 at one end and a sponge head 24 of relatively rigid sponge material attached to the other end of shaft 21, as illustrated in FIG. 1. When dry, the head 24 of this embodiment is a generally flat, part-circular or part disk shaped piece of surgical or medical grade sponge material such as relatively rigid, highly absorbent natural cellulose or highly absorbent, fast wicking polyvinyl acetate (PVA) material or the like, for example Ultracell® PVA or other sponge materials used in Weck-Cel® fluid control medical sponges as manufactured by Beaver-Visitec of Waltham, MA. The handle shaft 21 may be of any suitable material, such as injection molded plastic material, and may be transparent or opaque.

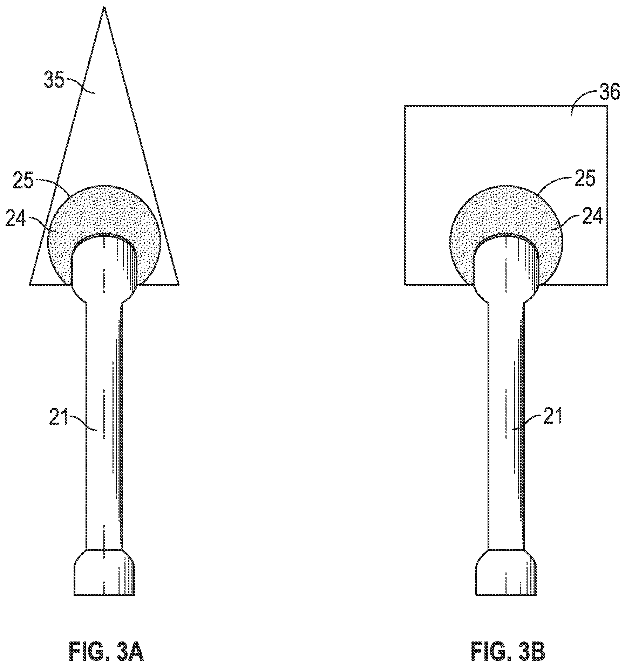

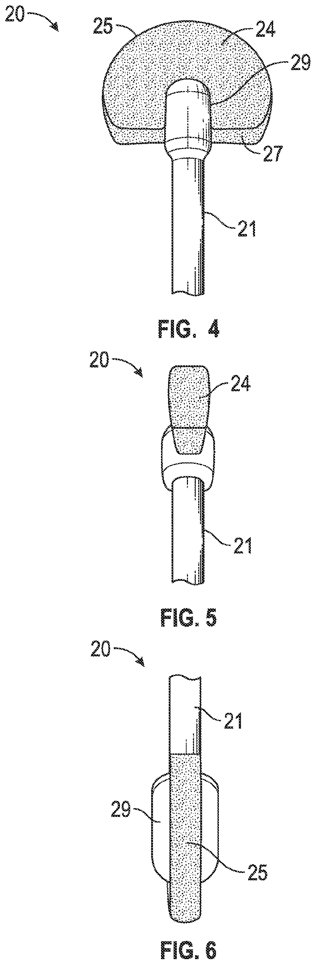

Sponge 24 has a rounded edge or rim 25 and a straight edge or rim 27, and is held at the center of the straight edge in recess 28 between spaced claws or end portions 29 of the shaft 21, as illustrated in FIG. 2. Sponge 24 may be secured in recess 28 via adhesive or the like. FIGS. 3A and 3B illustrate some alternative methods for manufacture of the preparation sponge device 20. The manufacturing method may be similar to that for conventional spear shaped medical sponges. In FIG. 3A, a spear shaped sponge 35 is secured to handle 21, and sponge 35 is then cut along line 25 to form part circular sponge 24. The thus-formed rounded edge or rim 25 forms more than half of the periphery of a circle, as can be seen in FIG. 3A. In FIG. 3B, a sponge blank 36 is attached to handle 21, and is cut along line 25 to form the part-circular sponge 24. In each case, the rounded edge 25 passes the half way or semi-circle point and starts to curve back in towards the handle, as illustrated in FIG. 1 and 4. The thickness of the sponge may be around 1 mm while the diameter of the part circular sponge may be in the range from 4.5 mm to around 8 mm. In one example, the diameter of the part-circular edge was about 6.5 mm.

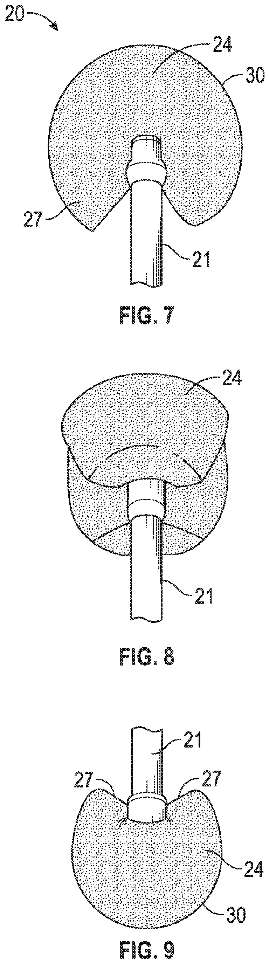

FIGS. 1, 2 and 4 to 6 illustrate the sponge device 20 from different directions, with the sponge 24 in a dry, unexpanded state. When the sponge is wetted, the sponge material swells and continues to swell until it creates a rounded ball-like or substantially spherical shape, as illustrated in FIGS. 7 to 9. The handle attachment at the center of the sponge restricts the central portion from swelling, while allowing the remainder of the sponge to swell or expand freely, creating eye preparation surfaces 30 which are all rounded when the sponge is sufficiently wetted, as seen in FIGS. 7 to 9. Because the sponge comprises more than half of a circle and the straight edge 27 of the sponge faces towards the handle when the device is in use, the straight edges on each side of the attachment area are moved away from the treatment area as the sponge device is wetted and expands, forming a generally V-shape as seen in FIG. 7 and 9. This moves edge surfaces away from the treatment area and avoids the risk of an edge surface contacting the eye.

In some embodiments, the sponge 24 is pre-wetted prior to use so that it is in the expanded condition of FIG. 7 to 9 before contacting the eye. Once wetted, the material has a softness and roundness that reduces the risk of causing epithelial defects when rubbing gently over the epithelial surface of the eye, as illustrated in FIG. 10. In other embodiments, the sponge may not be pre-wetted prior to use, but starts swelling and rounding up when touched to the tear meniscus from contact with eye fluids, so that it softens prior to the eye surface preparation procedure.

In some embodiments, rubbing or buffing the epithelium gently with the expanded sponge 24 removes lipids, mucus and microvilli as well as dead epithelial cells which would otherwise resist penetration of fluid through the epithelium. In some embodiments, the wet sponge is rubbed gently over the surface of the eye, for example in a circular pattern. In some embodiments, a topical anesthetic is applied to the eye before the application and use of the wet sponge.

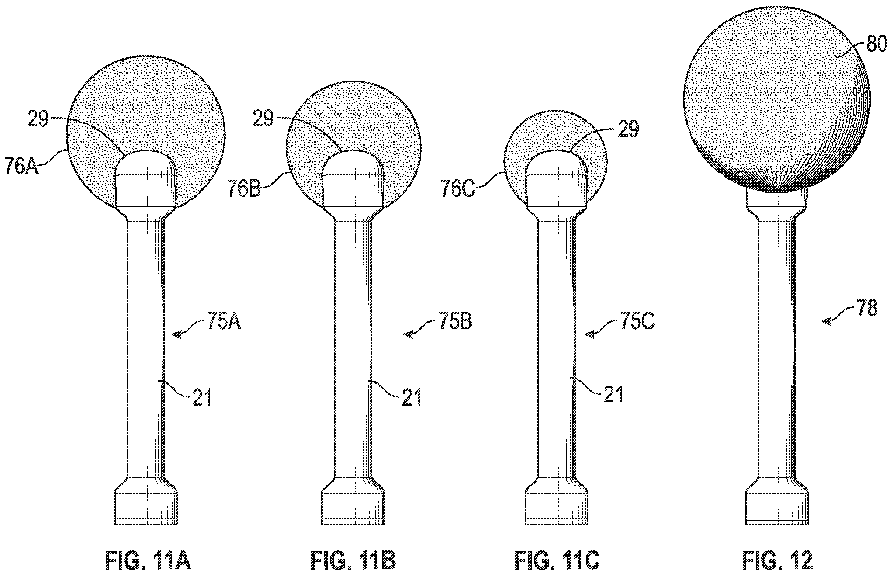

FIGS. 11A to 11C illustrate modified sponge devices 75A, 75B and 75C, respectively, which are similar to the sponge device of FIGS. 1 to 9, but have a completely circular head 76A, 76B, 76C attached to handle 21instead of a part circular head with a straight lower edge as in the first embodiment. In some embodiments, the head diameters in FIGS. 11A to 11C are around 8 mm, 6.5 mm, and 4.5 mm, respectively, and the dry sponge head has flat opposite faces as in the first embodiments. When wetted, the sponge heads of FIGS. 11A to 11C also expand to a substantially spherical shape. Modified sponge device 78 of FIG. 12 has a spherical head 80 of expanded foam material. This device is ready to use with a completely rounded surface, and absorbs liquid at a faster rate than the dry, compressed foam heads of the previous embodiments. Head 80 may be made of urethane sponge material, for example. This sponge device maintains structural rigidity for preparation of the epithelium for penetration of riboflavin solutions or other solutions or medications.

Any suitable sponge shape or material is contemplated for use with the methods disclosed herein, including the sponge head shape of the first embodiment (FIG. 1 to 10) as well as the alternative sponge head shapes of FIG. 11A to 12, as well as other shapes. In alternative embodiments, the preparation sponge device may be of more oval, Q-tip like shape, or may comprise a sponge or instrument wipe wrapped around a finger. However, the sponge head shape of FIG. 1 to 10 is found to work well in reducing the risk of causing injury or epi-defects while improving delivery of ophthalmic solution to the cornea, due to the generally rounded shape as well as the elimination of any sharp edges in the eye preparation surface portion when the sponge is wetted and expands as seen in FIG. 7 to 9. The sponge heads of FIG. 11A to 11C will also expand to a rounded, partially or completely spherical shape on wetting. The spherical sponge head of FIG. 12 is already completely expanded and rounded prior to use and absorbs liquid at a faster wicking rate than the heads of the other embodiments which are of dry compressed foam. The sponge heads in any of the preceding embodiments may be pre-wetted either prior to application to the eye or by placing on the eye surface to absorb tears, after which the head tends to swell or expand to a more rounded off shape with no sharp edges in the partially spherical tissue preparation surface.

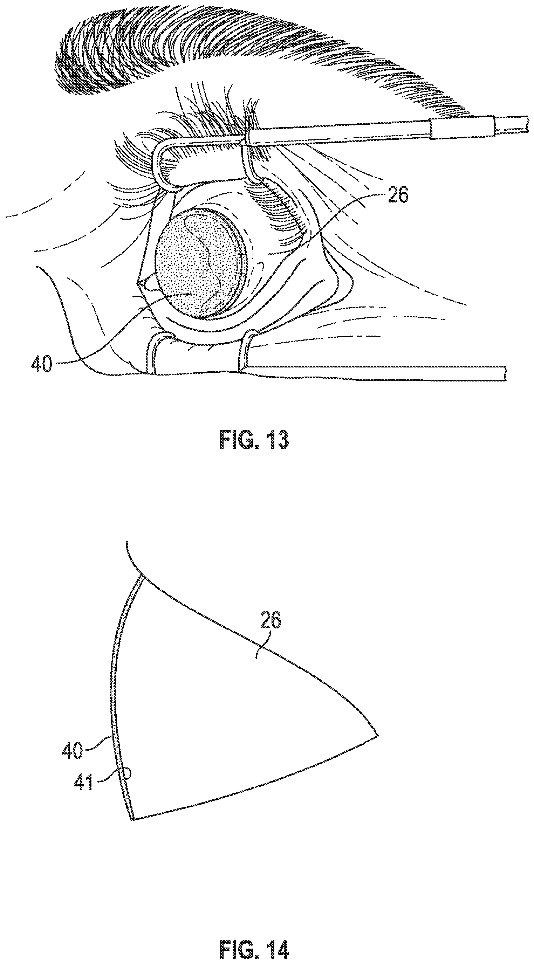

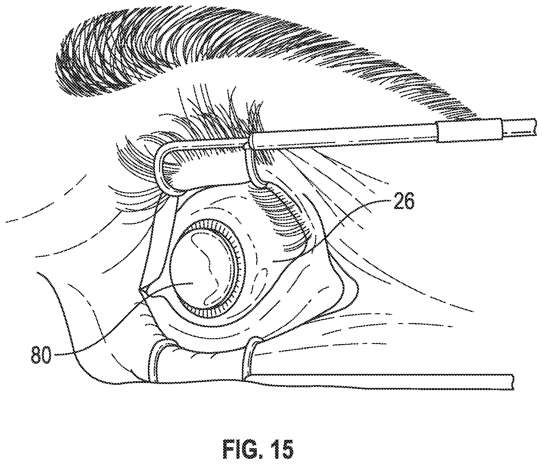

After completion of the polishing or buffing step, an ophthalmic solution is applied to the eye
through a second loading sponge device which comprises a solution holding or loading/moistening sponge 24 placed over the eye 26 as illustrated in FIGS. 13 and 14. Sponge 40 of FIG. 13 is circular and is thin enough to form to the curvature of the eye surface 41, as illustrated in FIG. 14. In some embodiments, solution holding or loading sponge 40 is of diameter large enough to cover or drape over all or most of the eye when centrally positioned as illustrated in FIG. 13 and 14, while in other embodiments, a smaller diameter loading sponge 80 which covers the corneal area of the eye only may be used, as illustrated in FIG. 15, depending on the desired treatment area. In the embodiment of FIG. 13 and 14, the sponge diameter is in the range from about 10 mm to about 12 mm, and has a thickness of the order of about 0.5 mm to about 3 mm. In one embodiment, the sponge diameter is about 11.5 mm. FIG. 15 illustrates smaller diameter solution holding or loading sponge 80 which covers at least the corneal area of the eye and may have a diameter in the range from about 3 mm to about 9 mm. In some embodiments, the sponges 40 and 80 are in the range of about 0.5 mm to about 5 mm in thickness. In some embodiments, the sponge becomes more flexible when soaked in ophthalmic solution so that it adopts the curvature of the eye when placed and thus contacts most of the area of the eye to allow the solution to penetrate through the epithelium into the cornea or other underlying regions of the eye. Sponge 40 may also be pre-formed in a dry state to conform or fit to the curvature of the eye surface. Although FIGS. 13 and 15 illustrate the eye being held open by a speculum during the solution loading procedure, this is often not necessary either for the eye preparation step of FIG. 12 or the loading procedure using sponge 40 or 80. In fact, the surgeon may hold the eye open manually during these procedures, or in some cases the patient may simply try not to blink.

In some embodiments, the ophthalmic solution is dripped onto the holding or loading sponge while it is placed over the eye, or the sponge is pre-soaked with the ophthalmic solution, or both. The second, loading sponge, for example sponge 40 or 80, acts as a reservoir to hold the solution against the eye surface and to allow application of additional solution. Simply dropping ophthalmic solution directly onto the eye surface or placing drops onto the eye results in the drops running off the eye, and has a limited effect. In contrast, the solution holding sponge of this embodiment places ophthalmic solution directly against the surface of the eye in a location where treatment is needed over an extended time period, allowing more efficient penetration into the cornea. In some embodiments, the ophthalmic solution is a riboflavin solution of 0.2% to 10.0% by weight riboflavin in an aqueous carrier, and optionally, sodium iodide, catalase, artificial tear solution or any combination thereof. If the sponge is pre-loaded with riboflavin, it is stored in a dark packaging material prior to use to avoid or reduce light degradation. In some embodiments, the riboflavin solution contains other additives for increased cross-linking, for example additives as described in PCT Application Publication No. WO 2013/148896. In some embodiments, the sponge is round, but the sponge may be of other shapes (such as oval or eye shaped) in alternative embodiments. In some embodiments, the solution holding or loading sponge operatively covers all or a portion of the eye or the cornea. In some embodiments, the holding sponge is made from any suitable sponge material such as cellulose or any fast wicking, lint-free material such as polyvinyl acetate, for example any of the materials described above in connection with the manipulation or preparation sponge devices of FIGS. 1 to 12. The sponge material of both the preparation sponge device 20 and the holding or loading sponge 40 in one embodiment was PVA sponge material with a pore size in the range of about 60 microns to about 1000 microns. In one embodiment, the pore size of both sponges was in the range from about 60 to about 120 microns. In one example, the pore size was about 60 microns. In one embodiment, the holding or loading sponge may have a larger pore size than the preparation sponge 24 in order to hold more liquid for corneal loading purposes.

Where the sponge is a round sponge, the sponge operatively covers all or a portion of the eye or the cornea. In some embodiments, the size of the sponge does not exceed the size of the eye or the cornea. In some embodiments, the diameter of the sponge is about 3 mm to about 12 mm. In some embodiments, the sponge is about 1 mm to about 5 mm in thickness. A series of different diameter loading sponges may be provided for selection by the physician depending on the desired treatment area.

The above sponges may be used in the two stage method described above for enhanced, faster penetration of any ocular treatment solution through the epithelium and into the cornea, while reducing or minimizing the risk of epithelial defects. The ophthalmic solution may be an ocular riboflavin solution for use in corneal treatment such as photochemical cross linking, or for other ophthalmic uses. In one embodiment, the method may be used for application of a riboflavin solution which contains 0.1 wt. % to 5.0 wt. % riboflavin in an aqueous carrier solution, or up to 10.0% in some cases. In some embodiments, the solution contains about 0.5 wt. % riboflavin. In other examples, the solution contains about 1.0 wt. % riboflavin or about 2.0 wt. % riboflavin. The higher concentration of riboflavin can increase corneal cross-linking if associated with higher amounts of oxygen in the cornea. The riboflavin solution may be stored in an actinic glass or UV and visible light protected plastic containers prior to use, to avoid activation of the riboflavin by ambient light.

The above devices and methods are designed to increase permeability of the epithelium layer with low or minimal epithelial defects resulting from the preparation or tissue manipulation step. Results of testing show that use of the sponge of FIGS. 1 to 9 with a part-circular edge, which expands to a part-spherical or ball-like shape when wetted, may eliminate or substantially eliminate epithelial defects. Similar results may be achieved with the sponges of FIGS. 11A to 11C with a completely circular edges, which also expand to a completely or partially spherical shape, as well as the sponge device of FIG. 12, which is pre-expanded to a spherical shape. Methods using the preparation sponge device of FIGS. 1 to 9 and the loading or holding sponge device of FIGS. 13 to 15 have also been found to significantly reduce the time needed for sufficient riboflavin solution to penetrate into and saturate the cornea, as compared to the time needed with no pre-treatment of the epithelium to increase permeability. Some examples are provided below.

### Example

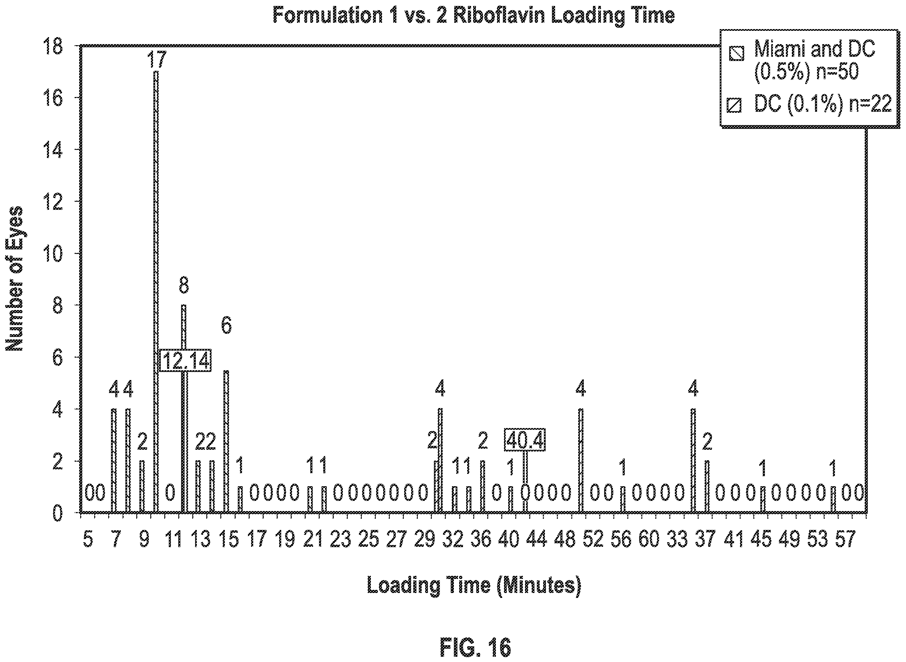

Two formulations of riboflavin solution were tested to determine saturation or riboflavin solution loading time after pre-treatment of the epithelium using the preparation sponge devices of the above embodiments. Formulation 1 had a riboflavin concentration of 0.1%. Formulation 2 had a riboflavin concentration of 0.5%. Results are compared in FIG. 16.

Saturation was determined using "serial slit-lamp assessments" of the cornea at approximately 5 minute intervals. Riboflavin has a characteristic green color when illuminated with visible light. Slit-lamp assessments using visible light reveal the depth and uniformity of riboflavin throughout the corneal thickness.

### Inclusion Criteria:

Patients who had undergone trans-epithelial cross-linking in one or both eyes were included in the analysis. Patients with a diagnosis of keratoconus or post-LASIK ectasia were included in this analysis.

### Exclusion Criteria:

Patients with previous RK, INTACS, more than one cross-linking procedure per eye, and/or patients who were pseudo-phakic or had a diagnosis of nuclear sclerotic cataract were excluded from this analysis

### Results-Formulation 1, 0.1% Riboflavin, Formulation 2, 0.5% Riboflavin

The results are shown in FIG. 16. Trans epithelial loading time using one of the loading sponges described above with formulation 1 ranged from 30 minutes minimum to 56 minutes maximum, with an average loading time of 40.4 minutes. Trans epithelial loading time with formulation 2 ranged from 7 to 30 minutes with an average loading time of 12.14 minutes.

The time period for good, homogeneous loading of riboflavin solution into the cornea, i.e. trans epithelial riboflavin loading time, was about 10 to 30 minutes using the delivery augmentation and delivery methods described above. Corresponding loading times without removal or pre-treatment of the epithelium can be up to three hours, significantly adding to the overall time for completion of a corneal treatment procedure.

## Claims

1. A delivery system for delivery of ophthalmic solutions to the eye, comprising:
a first, eye surface preparation sponge device comprising a handle and a head of absorbent, fast wicking sponge material secured to the handle, wherein the head is configured to expand from a first configuration having a first shape and dimensions when dry and a second configuration having a second shape and dimensions when wetted, at least the second configuration being generally rounded with no sharp edges and comprising an epithelial tissue preparation surface for rubbing across the surface of the epithelium of an eye to prepare the surface for penetration by ophthalmic solutions; and
a second, loading sponge device of absorbent, fast wicking sponge material comprising a circular, disc-shaped sponge of predetermined diameter which has increased flexibility when wetted and loaded with an ophthalmic solution, and is configured to cover at least the corneal portion of the eye and to adopt the curvature of the underlying eye surface when wetted with the ophthalmic solution and centrally placed over the eye for loading of ophthalmic solution into the eye after preparation of the epithelium with the first sponge device.

2. The delivery system of claim 1, further comprising a supply of ophthalmic solution for application to the epithelium.

3. The delivery system of claim 2 wherein the ophthalmic solution comprises 0.2% to 10.0% riboflavin in an aqueous carrier and sodium iodide.

4. The delivery system of claim 1, wherein the solution comprises 0.5% riboflavin.

5. The delivery system of claim 1, wherein the second, loading sponge device comprises a circular, disc-shaped sponge having a diameter in the range from about 8 mm to about 12 mm.

6. The delivery system of claim 1, wherein the head of the first sponge device has opposite flat surfaces and a peripheral rim of at least part circular shape in a dry condition and is configured to expand into a substantially ball-like shape when wet.

7. The delivery system of claim 6, wherein the peripheral rim of the head has a part-circular rim portion and a straight rim portion facing away from the part-circular portion in a dry condition, and the handle is secured at the center of the straight rim portion.

8. The delivery system of claim 6, wherein the thickness of the head in the dry condition is in the range from about 0.5 mm. to about 3 mm.

9. The delivery system of claim 8, wherein the thickness of the head in the dry condition is about 1 mm.

10. The delivery system of claim 8, wherein the thickness of the second sponge device in the dry condition is about the same as the thickness of the head of the first sponge device in the dry condition.

11. The delivery system of claim 6, wherein the diameter of the part-circular portion of the peripheral rim of the head in the dry condition is in the range from about 4.5 mm to around 8 mm.

12. The delivery system of claim 6, wherein the part-circular portion of the peripheral rim of the head in the dry condition extends around an arc greater than a semi-circle.

13. The delivery system of claim 12, wherein the straight rim portion is configured to form a V-shape when wetted with opposite parts of the V-shape extending on opposite sides of the handle.

14. The delivery system of claim 1, wherein the head of the first sponge device is of spherical shape.

15. The delivery system of claim 1, wherein the sponge material of both sponges has a pore size in the range from about 0.06 mm to about 1 mm, or in the range from about 0.06 mm to about 0.12 mm.

16. The delivery system of claim 14, wherein the material of the loading sponge device has a larger pore size than the material of the eye preparation sponge device.

17. The delivery system of claim 1, wherein the loading sponge device is pre-wetted with ophthalmic solution.

18. The delivery system of claim 16, wherein the sponge material of the first sponge device and the second sponge device is cellulose or polyvinyl acetate sponge material.

## Patentansprüche

1. Zuführsystem zum Zuführen von ophthalmischen Lösungen in das Auge, umfassend:
eine erste Schwammvorrichtung zur Augenoberflächenvorbereitung, umfassend einen Griff und einen an dem Griff befestigten Kopf aus saugfähigem Schwammmaterial mit schneller Dochtwirkung, wobei der Kopf dazu konfiguriert ist, sich von einer ersten Konfiguration mit einer ersten Form und ersten Abmessungen, wenn er trocken ist, und einer zweiten Konfiguration mit einer zweiten Form und zweiten Abmessungen, wenn er befeuchtet ist, auszudehnen, wobei mindestens die zweite Konfiguration allgemein abgerundet ohne scharfe Kanten ist, und umfassend eine Epithelgewebe-Vorbereitungsoberfläche zum Reiben über die Oberfläche des Epithels eines Auges, um die Oberfläche für die Penetration durch ophthalmische Lösungen vorzubereiten; und
eine zweite Schwammvorrichtung zum Einbringen aus saugfähigem Schwammmaterial mit schneller Dochtwirkung, umfassend einen kreisförmigen, scheibenförmigen Schwamm mit vorbestimmtem Durchmesser, der, wenn er befeuchtet ist und eine ophthalmische Lösung darin eingebracht ist, erhöhte Flexibilität aufweist, und dazu konfiguriert ist, mindestens den Hornhautabschnitt des Auges zu bedecken und die Krümmung der darunterliegenden Augenoberfläche anzunehmen, wenn er mit der ophthalmischen Lösung befeuchtet ist und mittig über das Auge platziert ist, um nach der Vorbereitung des Epithels mit der ersten Schwammvorrichtung die ophthalmische Lösung in das Auge einzubringen.

2. Zuführsystem nach Anspruch 1, weiter umfassend einen Vorrat an ophthalmischer Lösung zum Anwenden auf das Epithel.

3. Zuführsystem nach Anspruch 2, wobei die ophthalmische Lösung 0,2 % bis 10,0 % Riboflavin in einem wässrigen Träger und Natriumiodid umfasst.

4. Zuführsystem nach Anspruch 1, wobei die Lösung 0,5 % Riboflavin umfasst.

5. Zuführsystem nach Anspruch 1, wobei die zweite Schwammvorrichtung zum Einbringen einen kreisförmigen, scheibenförmigen Schwamm mit einem Durchmesser im Bereich von etwa 8 mm bis etwa 12 mm umfasst.

6. Zuführsystem nach Anspruch 1, wobei der Kopf der ersten Schwammvorrichtung in einem trockenen Zustand gegenüberliegende flache Oberfläche und einen Umfangsrand mit mindestens teilkreisförmiger Form aufweist und dazu konfiguriert ist, sich, wenn er nass ist, zu einer im Wesentlichen kugelförmigen Form auszudehnen.

7. Zuführsystem nach Anspruch 6, wobei der Umfangsrand des Kopfs in einem trockenen Zustand einen teilkreisförmigen Randabschnitt und einen in einem dem teilkreisförmigen Abschnitt abgewandten geraden Randabschnitt aufweist und der Griff in der Mitte des geraden Randabschnitts befestigt ist.

8. Zuführsystem nach Anspruch 6, wobei die Dicke des Kopfs im trockenen Zustand im Bereich von etwa 0,5 mm bis etwa 3 mm liegt.

9. Zuführsystem nach Anspruch 8, wobei die Dicke des Kopfs im trockenen Zustand etwa 1 mm beträgt.

10. Zuführsystem nach Anspruch 8, wobei die Dicke der zweiten Schwammvorrichtung im trockenen Zustand etwa gleich der Dicke des Kopfs der ersten Schwammvorrichtung im trockenen Zustand ist.

11. Zuführvorrichtung nach Anspruch 6, wobei der Durchmesser des teilkreisförmigen Abschnitts des Umfangsrands des Kopfs im trockenen Zustand im Bereich von etwa 4,5 mm bis ungefähr 8 mm liegt.

12. Zuführsystem nach Anspruch 6, wobei sich der teilkreisförmige Abschnitt des Umfangsrands des Kops im trockenen Zustand über einen Bogen erstreckt, der größer als ein Halbkreis ist.

13. Zuführsystem nach Anspruch 12, wobei der gerade Randabschnitt dazu konfiguriert ist, wenn er befeuchtet ist, eine V-Form zu bilden, wobei sich gegenüberliegende Teile der V-Form auf gegenüberliegenden Seiten des Griffs erstrecken.

14. Zuführsystem nach Anspruch 1, wobei der Kopf der ersten Schwammvorrichtung von allgemein sphärischer Form ist.

15. Zuführsystem nach Anspruch 1, wobei das Schwammmaterial beider Schwämme eine Porengröße im Bereich von etwa 0,06 mm bis etwa 1 mm oder im Bereich von etwa 0,06 mm bis etwa 0,12 mm liegt.

16. Zuführsystem nach Anspruch 14, wobei das Material der Schwammvorrichtung zum Einbringen eine größere Porengröße aufweist als das Material der Schwammvorrichtung zur Augenvorbereitung.

17. Zuführsystem nach Anspruch 1, wobei die Schwammvorrichtung zum Einbringen mit ophthalmischer Lösung vorbefeuchtet ist.

18. Zuführsystem nach Anspruch 16, wobei es sich bei dem Schwammmaterial der ersten Schwammvorrichtung und der zweiten Schwammvorrichtung um Cellulose- oder Polyvinylacetat-Schwammmaterial handelt.

## Revendications

1. Système d'administration servant à des fins d'administration de solutions ophtalmiques au niveau de l'oeil, comportant :
un premier dispositif à éponge de préparation de la surface de l'oeil comportant un manche et une tête en matière éponge absorbante et à effet capillaire rapide assujettie sur le manche, dans lequel la tête est configurée pour s'étendre d'une première configuration ayant une première forme et des premières dimensions quand elle est sèche à une deuxième configuration ayant une deuxième forme et des deuxièmes dimensions quand elle est mouillée, au moins la deuxième configuration étant généralement arrondie sans angles vifs et comportant une surface de préparation du tissu épithélial servant à frotter en travers de la surface de l'épithélium d'un oeil pour préparer la surface à des fins de pénétration par des solutions ophtalmiques ; et
un deuxième dispositif à éponge de charge en matière éponge absorbante et à effet capillaire rapide comportant une éponge circulaire en forme de disque d'un diamètre prédéterminé qui a une flexibilité accrue quand elle est mouillée et chargée d'une solution ophtalmique, et qui est configurée pour couvrir au moins la partie cornéenne de l'oeil et pour adopter la courbure de la surface sous-jacente de l'oeil quand elle est mouillée au moyen de la solution ophtalmique et placée centralement au-dessus de l'oeil à des fins de charge de la solution ophtalmique dans l'oeil après la préparation de l'épithélium au moyen du premier dispositif à éponge.

2. Système d'administration selon la revendication 1, comportant par ailleurs une alimentation en solution ophtalmique à des fins d'application sur l'épithélium.

3. Système d'administration selon la revendication 2, dans lequel la solution ophtalmique comporte de 0,2 % à 10,0 % de riboflavine dans un véhicule aqueux et de l'iodure de sodium.

4. Système d'administration selon la revendication 1, dans lequel la solution comporte 0,5 % de riboflavine.

5. Système d'administration selon la revendication 1, dans lequel le deuxième dispositif à éponge de charge comporte une éponge circulaire en forme de disque ayant un diamètre se trouvant dans la plage allant d'environ 8 mm à environ 12 mm.

6. Système d'administration selon la revendication 1, dans lequel la tête du premier dispositif à éponge a des surfaces plates opposées et un bord périphérique de forme au moins partiellement circulaire à l'état sec et est configurée pour s'étendre en une forme sensiblement similaire à une boule quand elle est mouillée.

7. Système d'administration selon la revendication 6, dans lequel le bord périphérique de la tête a une partie de bord partiellement circulaire et une partie de bord droite orientée à l'opposé de la partie partiellement circulaire à l'état sec, et le manche est assujetti au centre de la partie de bord droite.

8. Système d'administration selon la revendication 6, dans lequel l'épaisseur de la tête à l'état sec se trouve dans la plage allant d'environ 0,5 mm à environ 3 mm.

9. Système d'administration selon la revendication 8, dans lequel l'épaisseur de la tête à l'état sec mesure environ 1 mm.

10. Système d'administration selon la revendication 8, dans lequel l'épaisseur du deuxième dispositif à éponge à l'état sec est environ identique par rapport à l'épaisseur de la tête du premier dispositif à éponge à l'état sec.

11. Système d'administration selon la revendication 6, dans lequel le diamètre de la partie partiellement circulaire du bord périphérique de la tête à l'état sec se trouve dans la plage allant d'environ 4,5 mm à environ 8 mm.

12. Système d'administration selon la revendication 6, dans lequel la partie partiellement circulaire du bord périphérique de la tête à l'état sec s'étend autour d'un arc supérieur à un demi-cercle.

13. Système d'administration selon la revendication 12, dans lequel la partie de bord droite est configurée pour former une forme en V quand elle est mouillée avec des parties opposées de la forme en V s'étendant sur les côtés opposés du manche.

14. Système d'administration selon la revendication 1, dans lequel la tête du premier dispositif à éponge est d'une forme sphérique.

15. Système d'administration selon la revendication 1, dans lequel la matière éponge des deux éponges a un diamètre de pore se trouvant dans la plage allant d'environ 0,06 mm à environ 1 mm, ou dans la plage allant d'environ 0,06 mm à environ 0,12 mm.

16. Système d'administration selon la revendication 14, dans lequel la matière du dispositif à éponge de charge a un diamètre de pore supérieur par rapport à la matière du dispositif à éponge de préparation de l'oeil.

17. Système d'administration selon la revendication 1, dans lequel le dispositif à éponge de charge est pré-humidifié au moyen d'une solution ophtalmique.

18. Système d'administration selon la revendication 16, dans lequel la matière éponge du premier dispositif à éponge et du deuxième dispositif à éponge est une matière éponge d'acétate de cellulose ou de polyvinyle.
